# EUROPEAN PATENT APPLICATION

(11) **EP 4 736 911 A1**
(43) Date of publication of application: **06.05.2026**
(21) Application number: 24306818.6
(22) Date of filing: 29.10.2024
(51) Int. Cl.: A61M 5/315

(54) **METHOD FOR MANUFACTURING A PREFILLED SYRINGE TO REDUCE GLIDING FORCE THEREIN**

(71) Applicant: Becton Dickinson France, 38800 Le Pont-de-Claix (FR); Becton, Dickinson and Company, Franklin Lakes, NJ 07417 (US)
(72) Inventor: RIVE, Adrien, 38170 Seyssinet-Pariset (FR); MILLERET, Anne, 38530 Pontcharra (FR); VOIRIN, Coralie, 38360 Sassenage (FR); PERRIN, Eloïse, 38320 Eybens (FR); RODRIGUEZ SAN JUAN, Nestor, Hamburg, New Jersey 07419 (US)
(74) Representative: Germain Maureau

(57) **Abstract**

Provided herein is a method for manufacturing a prefilled syringe. The method includes providing a prefilled syringe including a syringe barrel defining a chamber containing a fluid therein and a stopper axially sealing the fluid in the chamber, with the stopper including one or more ribs forming a sealing surface with the syringe barrel. The method also includes performing a terminal steaming process on the prefilled syringe, with the terminal steaming process including a first steaming cycle that exposes the syringe to steam at a set temperature and a set pressure and a second steaming cycle that exposes the syringe to steam at the set temperature and the set pressure. Performing of the terminal steaming process reduces a diameter of at least one rib of the one or more ribs, to reduce a contact pressure between the stopper and the syringe barrel.

## Description

### BACKGROUND OF THE INVENTION

### Field of the Invention

The present disclosure relates generally to prefilled syringes and, more particularly, to a system and method for manufacturing prefilled syringes to reduce the gliding forces associated therewith.

### Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of an elastomeric material (preferably rubber, but possibly a thermoplastic elastomer) and is adapted to ensure the container closure integrity (CCI) of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The tail portion of the stopper may include a cavity formed therein configured to receive a distal head of the plunger rod, so as to engage the plunger rod with the stopper. Each of the cavity and the plunger head may be configured to provide engagement therebetween to secure the plunger rod to the stopper. The head portion of the stopper may include one or a plurality of annular, outwardly protruding ribs formed on an external cylindrical wall thereof, with the rib(s) applying a contact pressure against an inner surface of the syringe barrel to form a seal therewith that ensures the CCI of the syringe. A single rib or a series of two or three ribs may be spaced apart longitudinally along the stopper main body.

It is recognized that ensuring of the CCI of the syringe is vital during both transport/storage of the syringe and use of the syringe, with it being desired to maintain an adequate contact pressure between the stopper and the syringe barrel to ensure the CCI. However, while a higher contact pressure is desirable for ensuring of the CCI of the syringe, such high contact pressure increases the friction between the stopper and inner surface of the syringe barrel and makes it more difficult to move the stopper through the syringe barrel during use of the syringe, making the syringe less user friendly. That is, a high contact pressure between the stopper and the syringe barrel increases the activation and gliding forces required to initiate movement of the plunger assembly relative to the syringe barrel and to further advance the plunger assembly distally into the syringe barrel during use.

Accordingly, a need exists in the art for a prefilled syringe and method of manufacture thereof that provides sufficient contact pressure between the stopper and the syringe barrel, but that provides lower gliding forces during use of the prefilled syringe.

### SUMMARY OF THE INVENTION

Provided herein is a method for manufacturing a prefilled syringe. The method includes providing a prefilled syringe comprising a syringe barrel having a barrel proximal end and a barrel distal end and defining a chamber containing a fluid therein, and a stopper axially sealing the fluid in the chamber and movable within the chamber of the syringe barrel, the stopper including a main body and one or more ribs extending from an outer surface of the main body and around an outer circumference of the main body, the one or more ribs forming a sealing surface with the syringe barrel. The method also includes performing a terminal steaming process on the prefilled syringe, wherein performing the terminal steaming process further comprises performing one or more steaming cycles to expose the syringe to steam at the set temperature and the set pressure. Performing of the terminal steaming process reduces a diameter of at least one rib of the one or more ribs, to reduce a contact pressure between the stopper and the syringe barrel.

In certain configurations, performing the one or more steaming cycles comprises performing a single steaming cycle.

In certain configurations, performing the one or more steaming cycles comprises performing a first steaming cycle to expose the syringe to steam at a set temperature and a set pressure and performing a second steaming cycle to expose the syringe to steam at the set temperature and the set pressure.

In certain configurations, in performing the one or more steaming cycles, the syringe is exposed to steam at a temperature of 90-200° C.

In certain configurations, in performing the one or more steaming cycles, the syringe is exposed to steam at a pressure of at least 2 bars and preferably 3 bars or more.

In certain configurations, the one or more steaming cycles each have a duration of 10 minutes or more, preferably 20 minutes or more, more preferably 30 minutes or more, and most preferably 35 minutes or more.

In certain configurations, performing the one or more steaming cycles comprises performing a cooling with ventilation.

In certain configurations, the cooling with ventilation is performed at a temperature of 50° C and at a pressure of 3 bars.

In certain configurations, in performing the terminal steaming process, the diameter of the at least one rib is reduced by at least 0.1 mm, and preferably 0.1 to 0.15 mm.

In certain configurations, the gliding force required to advance the stopper within the syringe barrel is reduced by 30-60%, and preferably 40-45%, after performing of the terminal steaming, as compared to no terminal steaming being performed.

In certain configurations, performing of the terminal steaming process compression sets an elastomeric material of the stopper, to reduce the diameter of the at least one rib.

Also provided herein is a syringe comprising a syringe barrel and a stopper positioned therein. The stopper is prepared for sliding engagement with the syringe barrel by performing a terminal steaming process on the prefilled syringe, wherein performing the terminal steaming process further comprises performing a first steaming cycle to expose the syringe to steam at a set temperature and a set pressure and performing a second steaming cycle to expose the syringe to steam at the set temperature and the set pressure. Performing of the terminal steaming process reduces a diameter of at least one rib of the one or more ribs, to reduce a contact pressure between the stopper and the syringe barrel.

In certain configurations, the stopper comprises at least one rib, and wherein performing of the terminal steaming process reduces the diameter of the at least one rib.

In certain configurations, the syringe barrel comprises one of a siliconized glass barrel, a non-siliconized glass barrel, or a plastic barrel with or without a silicone coating.

In certain configurations, the stopper comprises a coated stopper or an uncoated stopper.

In certain configurations, the stopper is formed of butyl rubber, styrene butadiene rubber, poly isoprene rubber, liquid silicone rubber, or a thermoplastic elastomer, and when the stopper comprises a coated stopper, the coating comprises silicone oil, PTFE, ETFE, polyethylene, parylene, or liquid silicone rubber.

### BRIEF DESCRIPTION OF THE DRAWINGS

FIG. 1 is a perspective view of a syringe, with which embodiments of the disclosure may be implemented;
FIG. 2 is an exploded view of the syringe of FIG. 1;
FIG. 3 is a side view of the stopper included in the syringe of FIG. 1, according to a non-limiting embodiment described herein;
FIG. 4 is a flowchart illustrating a method for manufacturing a prefilled syringe to reduce the gliding forces associated therewith, according to a non-limiting embodiment described herein;
FIG. 5 is a graph illustrating a diameter of a rib on the stopper for treated and untreated syringes and over various ageing periods, according to a non-limiting embodiment described herein; and
FIG. 6 is a graph illustrating activation and gliding forces for treated and untreated syringes, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

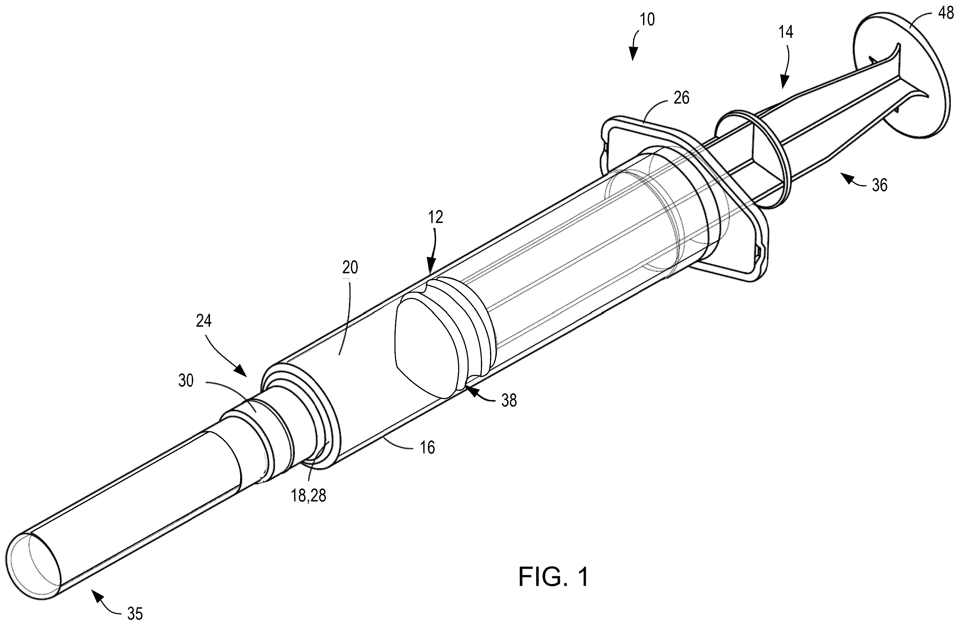

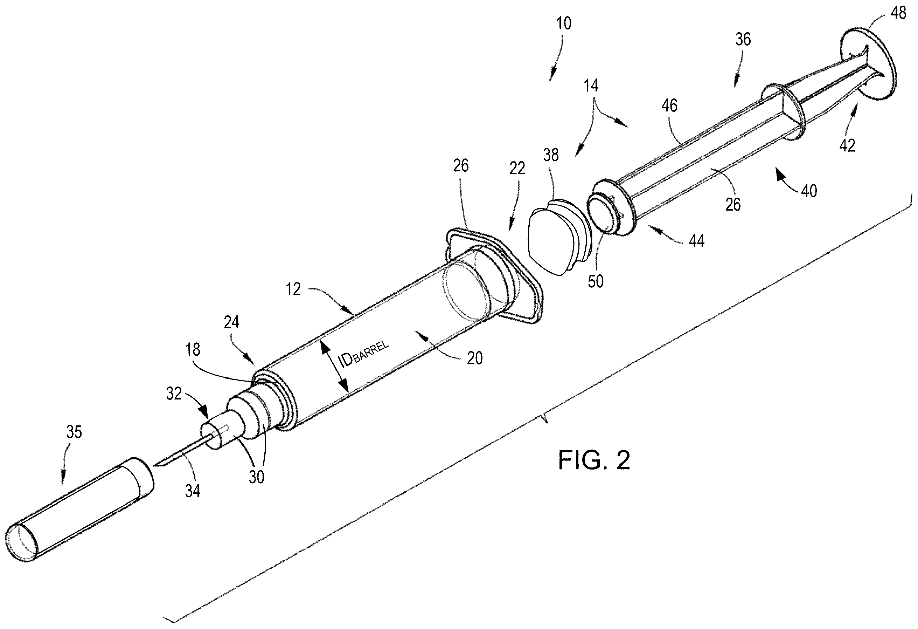

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein, with the syringe barrel 12 formed of glass or plastic, according to various embodiments. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

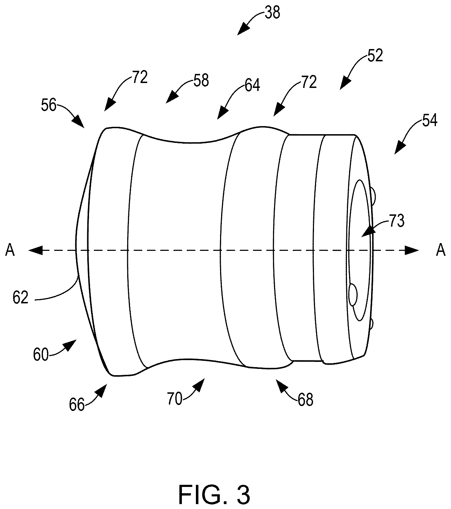

The plunger assembly 14 of syringe 10 is formed of an elongate plunger rod 36 and a plunger head or stopper 38. The plunger rod 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 12. In some embodiments, the plunger distal end 44 may include a threaded extension member 50, or alternately a hook, that extends out and is configured to mate with the stopper 38, such as via a twisting engagement with a threaded inner cavity 73 (FIG. 3) of the stopper 38.

The stopper 38 of plunger assembly 14 is positioned at the plunger distal end 44 so as to be movable along with the plunger rod 36 within the chamber 20 of syringe barrel 12. The stopper 38 may be made from a material that is different from the material of the plunger rod 36 and that is capable of forming a tight seal with the syringe barrel 12 as it is advanced therethrough. In some embodiments, the stopper 38 is formed of an elastomeric material such as butyl rubber, styrene butadiene rubber, poly isoprene rubber, or liquid silicone rubber, as non-limiting examples. In other embodiments, the stopper 38 may instead be formed of a polymeric material, such as crosslinked or thermoplastic elastomers, as non-limiting examples.

Referring now to FIG. 3, and with continued reference to FIGS. 1 and 2, the structure of stopper 38 is shown in greater detail, according to an aspect or embodiment of the disclosure. The stopper 38 is defined by a main body 52 that includes an open proximal end 54 that engages with the distal end of plunger rod 36 and a closed distal end 56 configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

An outer surface 64 of the main body 52 includes a plurality of ribs 66, 68 formed thereon that extend circumferentially around the entire outer surface 64. In the illustrated embodiment, the plurality of ribs 66, 68 includes a first rib 66 and a second rib 68, although it is recognized that the stopper 38 could include a lesser number of ribs (i.e., one) or a greater number of ribs, such as three or four ribs. The first and second ribs 66, 68 are spaced apart longitudinally, such that the first rib 66 is positioned toward the proximal end 54 of the main body 52 and the second rib 68 is positioned toward the distal end 56 of the main body 52, with an inter-rib region 70 present between the first and second ribs 66, 68. Ribs 66, 68 are thus referred to as "distal rib 66" and "proximal rib 68." The structure of each of distal rib 66 and proximal rib 68 may be controlled to achieve a desired interaction between the stopper 38 and the barrel 12. According to one aspect of ribs 66, 68, the ribs 66, 68 extend radially outward a desired distance from the outer surface 64 (i.e., a rib thickness) to act as bearing points that provide a contact pressure against the inner surface of the syringe barrel 12.

According to some aspects of the disclosure, the stopper 38 may further include one or more outer coatings or layers (not shown) applied to portions thereof, such as on a sealant or gliding surface thereof - i.e., on a contact surface 72 of distal rib 66 and proximal rib 68. The coating may be configured to reduce friction between the stopper 38 and barrel 12 when the stopper 38 is moved within the syringe 10. According to non-limiting embodiments, the coating may be provided as silicone oil, PTFE, ETFE, polyethylene, parylene, liquid silicone rubber, or another suitable coating that reduces friction and/or reduces the interaction between the stopper 38 and the medication/drug in the syringe barrel 12 (i.e., extractables and leachables).

With the construction of the syringe 10 as described above, it is desirable for the syringe 10 to be configured to maintain CCI in the syringe 10, while also providing desirable gliding forces during use of the syringe 10. That is, it is desirable for the stopper 38 to apply a radially outward pressure against the syringe barrel 12 in order to maintain CCI in the syringe 10 during transport/storage and use, but it is also desirable to control gliding forces needed to advance the plunger assembly 14 during use of the syringe 10. According to aspects of the disclosure, a method for manufacturing the syringe 10 is provided that aids in controlling the gliding forces needed to advance the plunger assembly 14 during use of the syringe 10. That is, a steaming process is performed on the stopper 38 to provide for controlling of such gliding forces. The steaming process may be performed to "treat" the stopper 38, which reduces the dimensions of the ribs/main body thereof, thereby reducing the contact pressure between the stopper 38 and the syringe barrel 12 so as to lower the gliding forces needed to move the stopper 38 within the syringe barrel 12.

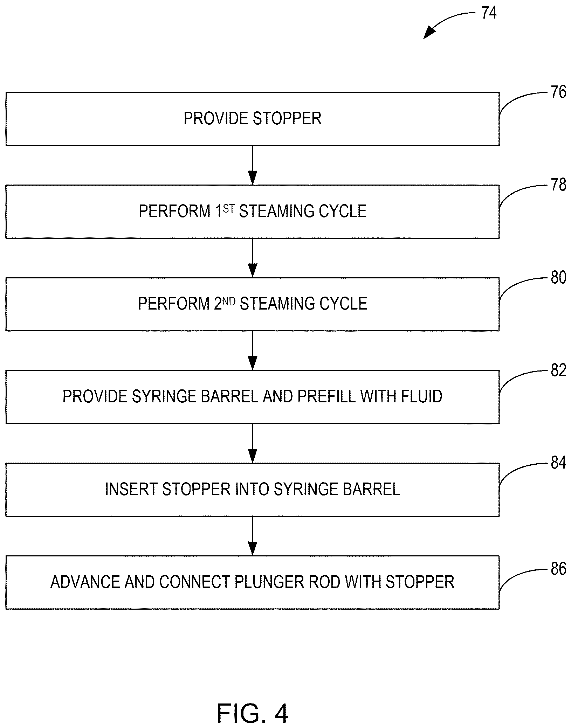

Referring now to FIG. 4, and with continued reference to FIGS. 1-3, a flowchart illustrating a method 74 for manufacturing a prefilled syringe (e.g., syringe 10) is provided, according to an embodiment of the disclosure.

The method 74 may begin by providing a stopper 38 at step 76. As previously described, the stopper 38 may be provided as an uncoated stopper (formed only of a base, elastomeric material such as butyl rubber, styrene butadiene rubber, poly isoprene rubber, liquid silicone rubber, as non-limiting examples) or may be provided as a coated stopper, where an outer coating (or layer) is applied over the base material, with exemplary coatings being silicone oil, PTFE, ETFE, polyethylene, parylene, liquid silicone rubber, or another suitable coating that reduces friction and/or reduces the interaction between the stopper 38 and the medication/drug in the syringe barrel 12 (i.e., extractables and leachables).

According to aspects of the disclosure, the stopper 38 is then subjected to a terminal steaming process at steps 78 and 80. According to an exemplary embodiment, the terminal steaming is performed as a two-step process - including a first steaming cycle (step 78) and a second steaming cycle (step 80). While not shown in the method 74 of FIG. 4, it is recognized that in other embodiments, the terminal steaming process may instead include only a single steaming cycle.

As indicated in FIG. 4, a first steaming cycle is performed at step 78. In performing the first steaming cycle, the stopper 38 is exposed to a source of heat, such as saturated steam. In some embodiments, the stopper 38 may be placed in an autoclave with the temperature set between 90° C and 200° C, and preferably at 121° C, and the pressure being set at 2 bars or more and preferably 3 bars or more. The pressurized, saturated steam heats the stopper 38 for a predetermined length of time, with the first steaming cycle lasting for 10 minutes or more, preferably 20 minutes or more, more preferably 30 minutes or more, and most preferably 35 minutes or more, according to an exemplary embodiment. After expiration of the predetermined time for applying the saturated steam to the stopper 38, the first steaming cycle nay be completed by cooling the stopper 38, such as via cooling with ventilation at a temperature of 50° C and at a pressure of 3 bars.

After completion of the first steaming cycle at step 78 (and any optional intervening waiting period), a second steaming cycle is performed at step 80. The second steaming cycle may be similar or identical to the first steaming cycle regarding parameters of steam temperature and pressure. Thus, in some embodiments, the stopper 38 may be placed in an autoclave with the temperature set between 90° C and 200° C, and preferably at 121° C, and the pressure being set at 2 bars or more and preferably 3 bars or more. The pressurized, saturated steam heats the stopper 38 for a predetermined length of time, with the second steaming cycle lasting for 10 minutes or more, preferably 20 minutes or more, more preferably 30 minutes or more, and most preferably 35 minutes or more, according to an exemplary embodiment. After expiration of the predetermined time for applying the saturated steam to the stopper 38, the second steaming cycle may be completed by cooling the stopper 38, such as via cooling with ventilation at a temperature of 50° C and at a pressure of 3 bars.

The method 74 may continue with providing a syringe barrel 12 and filling the syringe barrel 12 with a medicament or other fluid at step 82. According to embodiments, the syringe barrel 12 may be provided as a siliconized glass barrel, a non-siliconized glass barrel, or a plastic barrel with or without a silicone coating. In filling the syringe barrel 12 with a medicament or other fluid, a controlled amount and type of fluid may be dispensed into syringe barrel 12, as dictated by the intended end-use of the syringe 10.

Upon filling of the syringe barrel 12 with a fluid, the method continues at step 84 by inserting the stopper 38 into the syringe barrel 12 and positioning the stopper 38 at a desired location therein. The inserting/positioning of stopper 38 within syringe barrel 12 may be performed by a known process, such as vent tube stoppering for example. The vent tube stoppering may be performed via use of a stoppering rod, as known in the art, and prior to engagement of plunger rod 36 with stopper 38.

In some embodiments, after positioning of the stopper 38 within syringe barrel 12 at a desired location, the method 74 may continue at step 86 by advancing the plunger rod 36 into the syringe barrel 12 and connecting the plunger rod 36 with the stopper 38. In some embodiments, a threaded extension member 50 of plunger rod 36 may be screwed into a threaded inner cavity 73 of the stopper 38, to secure the plunger rod 36 to the stopper 38, while in other embodiments a hook of plunger rod 36 may be engaged in a cavity of the stopper 38 to secure the plunger rod 36 to the stopper 38.

According to aspects of the disclosure, performing of the method 74 as described above provides a manufactured syringe 10 having acceptable gliding force levels. The terminal steaming process performed as part of the method "treats" the stopper 38, which reduces the dimensions of one or more of the ribs 66, 68 thereof, thereby reducing the contact pressure between the stopper 38 and the syringe barrel 12 so as to lower the gliding forces needed to move the stopper 38 within the syringe barrel 12. That is, performing of the steaming process at steps 78 and 80 functions to compression set the stopper 38 (i.e., the base elastomeric material forming the stopper), so as to reduce the dimensions/diameters of one or more of the ribs 66, 68 thereof. The reducing of the dimensions/diameters of one or more of the ribs 66, 68 reduces a contact pressure between the stopper 38 and the syringe barrel 12, thereby also lowering the gliding forces needed to move the stopper 38 within the syringe barrel 12. Beneficially, the reduction of the dimensions/diameters of one or more of the ribs 66, 68 of stopper 38 is retained over time, with no subsequent changes in the dimensions/diameters of the rib(s) 66, 68 being observed over time.

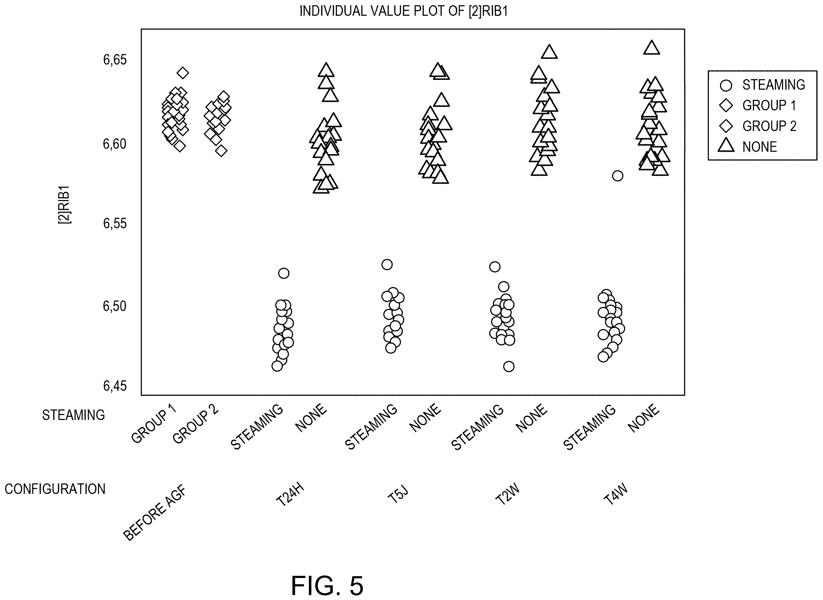

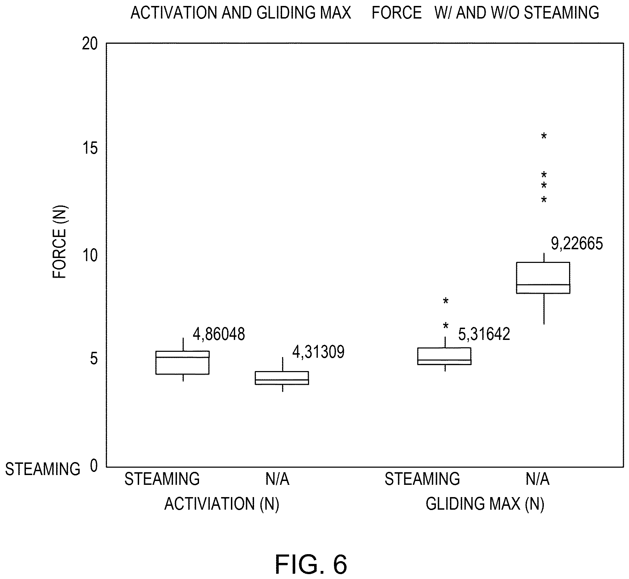

An example of the reducing of the dimensions/diameters of one or more of the ribs 66, 68 of the stopper 38 and the associated lowered gliding forces observed in syringe 10 that is observed following performing of the terminal steaming process is provided in the graphs of FIGS. 5 and 6. The results illustrated are for a syringe 10 having a siliconized syringe barrel 12 and a stopper 38 partially laminated/coated with ETFE, but it is recognized that similar results could be achieved for syringes with a non-siliconized syringe barrel 12 and/or an uncoated stopper 38.

Referring first to FIG. 5, a graph is provided therein showing a diameter of a rib (e.g., first rib 66) when stoppered in syringe barrel 12, at different points in time (i.e., ageing periods of 24 hours, 5 days, 2 weeks, and 4 weeks) and with/without exposure of the stopper 38 to the steaming process described above. As indicated therein, the first rib 66 has an initial diameter of approximately 6.60-6.65 mm. This initial diameter of the first rib 66 does not change substantially if the stopper 38 is not exposed to the steaming process, with the diameter remaining at approximately 6.55-6.65 mm over all ageing periods. However, after exposure of the stopper 38 to the steaming process, the diameter of the first rib 66 is reduced from its initial diameter (6.60-6.65 mm) to a reduced diameter of approximately 6.47-6.52 mm - i.e., a reduction in diameter of 0.1-0.15 mm. As seen in the graph, the reduced diameter of the stopper 38 is maintained at approximately 6.47-6.52 mm over all ageing periods, with the compression setting of the stopper material keeping the diameter of the first rib 66 unchanged over time.

With the dimensions/diameter of the stopper 38 (i.e., of rib(s) 66, 68) being reduced as described above, it is recognized that the gliding force (and activation force) required to move the stopper 38 within the syringe barrel 12 may also be lowered, with the gliding force being substantially reduced. Referring now to FIG. 6, a graph is provided therein showing an activation force and a gliding force for the stopper 38, with and without exposure of the stopper 38 to the terminal steaming process described above. As indicated therein, for a syringe 10 having a siliconized syringe barrel 12 and a stopper 38 partially laminated/coated with ETFE, the activation force for initiating movement of the stopper 38 remains relatively unchanged for a stopper 38exposed to the terminal steaming process as compared to a stopper 38 not exposed to the terminal steaming process (i.e., 4.86 N vs. 4.31 N, respectively). However, the gliding force for distally advancing the stopper 38 is significantly less, such as 40-45% less, for a stopper 38 exposed to the terminal steaming process as compared to a stopper 38 not exposed to the terminal steaming process (i.e., 5.31 N vs. 9.22 N, respectively). Thus, for the specific configuration of the syringe 10 represented in FIGS. 5 and 6, it can be seen that the gliding force required for distally advancing the stopper 38 within syringe barrel 12 can be significantly reduced by exposure of the stopper 38 to a terminal steaming process.

Beneficially, embodiments of the disclosure thus are directed to a method for manufacturing a prefilled syringe having a syringe barrel and stopper with the method including a terminal steaming process that reduces a diameter of at least one circumferential rib on the stopper, to reduce a contact pressure between the stopper and the syringe barrel. By reducing the contact pressure, a gliding force for moving the stopper within the syringe barrel is reduced, so as to improve a user experience when using the syringe.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A method for manufacturing a prefilled syringe, the method comprising:
providing a prefilled syringe comprising:
a syringe barrel having a barrel proximal end and a barrel distal end and defining a chamber containing a fluid therein; and
a stopper axially sealing the fluid in the chamber and movable within the chamber of the syringe barrel, the stopper including a main body and one or more ribs extending from an outer surface of the main body and around an outer circumference of the main body, the one or more ribs forming a sealing surface with the syringe barrel; and
performing a terminal steaming process on the prefilled syringe, wherein performing the terminal steaming process comprises performing one or more steaming cycles to expose the syringe to steam at a set temperature and a set pressure;
wherein performing of the terminal steaming process reduces a diameter of at least one rib of the one or more ribs, to reduce a contact pressure between the stopper and the syringe barrel.

2. The method of claim 1, wherein performing the one or more steaming cycles comprises performing a single steaming cycle.

3. The method of claim 1, wherein performing the one or more steaming cycles comprises:
performing a first steam sterilization cycle to expose the syringe to steam at a set temperature and a set pressure; and
performing a second steam sterilization cycle to expose the syringe to steam at the set temperature and the set pressure.

4. The method of any of claims 1-3, wherein in performing the one or more steaming cycles, the syringe is exposed to steam at a temperature of 90-200° C .

5. The method of any of claims 1-4, wherein in performing the one or more steaming cycles, the syringe is exposed to steam at a pressure of at least 2 bars and preferably 3 bars or more.

6. The method of any of claims 1-5, the one or more steaming cycles each have a duration of 10 minutes or more, preferably 20 minutes or more, more preferably 30 minutes or more, and most preferably 35 minutes or more.

7. The method of any of claims 1-6, wherein performing the one or more steaming cycles comprises performing a cooling with ventilation.

8. The method of claim 6, wherein the cooling with ventilation is performed at a temperature of 50° C and at a pressure of 3 bars.

9. The method of any of claims 1-8, wherein in performing the terminal steaming process, the diameter of the at least one rib is reduced by at least 0.1 mm, and preferably 0.1 to 0.15 mm.

10. The method of any of claims 1-9, wherein the gliding force required to advance the stopper within the syringe barrel is reduced by30-60%, and preferably 40-45%, after performing of the terminal steaming, as compared to no terminal steaming being performed.

11. The method of any of claims 1-10, wherein performing of the terminal steaming process compression sets an elastomeric material of the stopper, to reduce the diameter of the at least one rib.

12. A syringe comprising a syringe barrel and a stopper positioned therein, wherein the stopper is prepared for sliding engagement with the syringe barrel by the method of claim 1.

13. The syringe of claim 12, wherein the stopper comprises at least one rib, and wherein performing of the terminal steaming process reduces the diameter of the at least one rib.

14. The syringe of claim 12 or claim 13, wherein the syringe barrel comprises one of a siliconized glass barrel, a non-siliconized glass barrel, or a plastic barrel with or without a silicone coating.

15. The syringe of any of claims 12-14, wherein the stopper comprises a coated stopper or an uncoated stopper.

16. The syringe of claim 15, wherein the stopper is formed of butyl rubber, styrene butadiene rubber, poly isoprene rubber, liquid silicone rubber, or a thermoplastic elastomer, and wherein when the stopper comprises a coated stopper, the coating comprising silicone oil, PTFE, ETFE, polyethylene, parylene, or liquid silicone rubber.
